# EUROPEAN PATENT APPLICATION

(11) **EP 4 678 186 A1**
(43) Date of publication of application: **14.01.2026**
(21) Application number: 24766505.2
(22) Date of filing: 07.03.2024
(51) Int. Cl.: A61K 39/395, A61P 35/00, A61P 35/02, A61K 31/47

(54) **PHARMACEUTICAL COMPOSITION COMPRISING ANTI-CTLA4-ANTI-PD-1 BISPECIFIC ANTIBODY AND USE THEREOF**

(30) Priority: 07.03.2023 CN 202310213266
(71) Applicant: Akeso Pharma Co., Ltd., Guangzhou, Guangdong 510555 (CN)
(72) Inventor: XIA, Yu, Zhongshan, Guangdong 528437 (CN); WANG, Zhongmin, Zhongshan, Guangdong 528437 (CN); LI, Baiyong, Zhongshan, Guangdong 528437 (CN)
(74) Representative: Berggren Oy
(86) International application number: PCT/CN2024/080526
(87) International publication number: WO 2024/183791

(57) **Abstract**

A pharmaceutical composition comprising an anti-CTLA4-anti-PD-1 bispecific antibody, comprising an anti-CTLA4-anti-PD-1 bispecific antibody, gemcitabine, and optionally paclitaxel (e.g., albumin-bound paclitaxel). The pharmaceutical composition has a good anti-tumor effect

## Description

### CROSS-REFERENCE TO RELATED APPLICATION

The present application is based on and claims priority to the Chinese Patent Application No. CN202310213266.3 filed on March 7, 2023, the content of which is incorporated herein by reference in its entirety.

### TECHNICAL FIELD

The present disclosure relates to the field of tumor treatment and molecular immunology, and to a therapeutic combination comprising an anti-CTLA4-anti-PD-1 bispecific antibody and use thereof. Specifically, the present disclosure relates to a therapeutic combination comprising an anti-CTLA4-anti-PD-1 bispecific antibody, gemcitabine, and paclitaxel (e.g., albumin-bound paclitaxel), and use thereof.

### BACKGROUND

The transmembrane receptor PD-1 (programmed cell death protein-1) is a member of the CD28 gene family, and is expressed in activated T cells, B cells, and myeloid cells. Ligands of PD-1, both PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells and epithelial cells, and PDL2 is expressed only in antigen-presenting cells such as dendritic cells and macrophages.

The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, play a role in killing tumor cells, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is the *in-vivo* injection of an anti-PD-1 antibody to modulate the expression of PD-1. Due to the broad-spectrum anti-tumor prospects and surprising efficacy of PD-1 antibodies, it is widely accepted in the industry that antibodies targeting the PD-1 pathway will bring about breakthroughs in the treatment of various tumors, for example, non-small cell lung cancer, renal cell carcinoma, ovarian cancer, melanoma (Homet M. B., Parisi G., et al., 2015, Semin Oncol., 42(3): 466-473), leukemia and anemia (Held SA, Heine A, et al., 2013, Curr Cancer Drug Targets., 13(7): 768-74).

Cytotoxic T lymphocyte-associated antigen 4 (CTLA4) and CD28 molecules are very similar in aspects of gene structure, chromosome location, sequence homology and gene expression. Both molecules are receptors of the co-stimulatory molecule B7, and are mainly expressed on the surface of activated T cells. Binding of CTLA4 to B7 inhibits the activation of mouse and human T cells, and plays a negative regulatory role in T cell activation.

CTLA4 antibodies (or anti-CTLA4 monoclonal antibodies) or CTLA4 ligands can prevent CTLA4 from binding to its natural ligands, thereby blocking the transmission of negative regulatory signals by CTLA4 to T cells and enhancing the reactivity of T cells to various antigens. In this respect, *in-vivo* and *in-vitro* studies are essentially consistent. Currently, there are CTLA4 monoclonal antibodies in clinical trials or approved for treating prostate cancer, bladder cancer, colorectal cancer, gastrointestinal cancer, liver cancer, malignant melanoma, etc. (Grosso JF., Jure-Kunkel MN., 2013, Cancer Immun., 13:5)*.*

Interleukin 2 (IL-2) is produced by T cells. It is a growth factor that regulates T cell subgroups, and is an important factor in regulating immune responses. It promotes the proliferation of activated B cells, and participates in antibody responses, hematopoiesis and tumor surveillance. Recombinant human IL-2 has been approved by the U.S. FDA for treating malignant tumors, including melanoma, renal tumors, etc., while a clinical study is currently ongoing for treating chronic viral infections (Chavez, A.R., et al., 2009, Ann. N.Y. Acad. Sci., 1182:p.14-27). CTLA4 and CTLA4 antibodies are important influencing factors of T cell functions and interfere with the immune microenvironment in the body. *In-vitro* and *in-vivo* studies demonstrated that CTLA4 antibodies can specifically relieve the immunosuppression of CTLA4, activate T cells, and induce IL-2 generation, and are promising in wide applications in gene therapy against diseases such as tumors and parasite infections.

CTLA4 antibodies can produce a specific therapeutic effect on diseases and show remarkable efficacy, and may be used for supplementing traditional medicines and for exploring new means of gene therapy.

Bispecific antibodies, also known as bifunctional antibodies, are specific drugs that target two different antigens simultaneously, and can be purified and produced by immunomagnetic separation. Alternatively, they can be obtained by genetic engineering. The genetic engineering has corresponding flexibility in aspects of binding site optimization, synthetic form consideration, yield and the like, thus having certain advantages. Currently, over 45 existence forms have been demonstrated (Dafne Muller, Kontermann R E., 2010, BioDrugs, 24(2): 89-98). A number of developed bispecific antibodies are in the form of IgG-scFv, i.e., the Morrison format (Coloma MJ, Morrison SL., 1997, Nat Biotechnol., 15: 159-163), which has been demonstrated to be one of the ideal forms for the bifunctional antibodies because of its similarity to the naturally existing IgG forms and advantages in antibody engineering, expression and purification (Miller BR, Demarest SJ, et al., 2010, Protein Eng Des Sel, 23: 549-57; Fitzgerald J, Lugovskoy A., 2011, MAbs, 3: 299-309).

Gemcitabine is a commonly used first-line chemotherapeutic drug for treating various solid tumors such as non-small cell lung cancer, pancreatic cancer, breast cancer, and bladder cancer, and can also be used for treating malignant lymphoma, ovarian cancer, and nasopharyngeal cancer. This drug is a difluoronucleoside antimetabolite anti-tumor drug, which inhibits the proliferation and metastasis of tumor cells by inhibiting the synthesis of deoxyribonucleic acid and interfering with the replication and transcription of cellular DNA. However, gemcitabine monotherapy for cancer has a limited effect, with its most significant disadvantage being a short half-life, and only when a large dose is continuously administered intravenously can an effective concentration be maintained, so that serious toxic and side effects are inevitably caused, and the quality of life of patients is significantly reduced. The chemical structural formula of gemcitabine is shown in formula I below. Gemcitabine hydrochloride is commonly used clinically, and its structural formula is shown in formula II below.

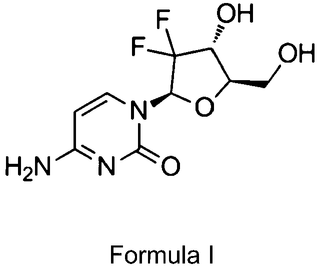

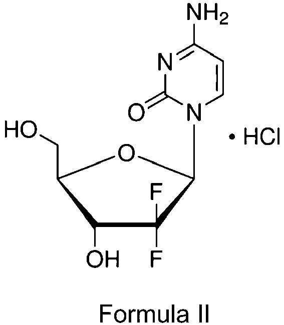

Paclitaxel is a natural plant-based new anti-tumor drug, and is a monomeric diterpenoid compound extracted from the bark of *Taxus brevifolia* by the National Cancer Institute (NCI) in the late 1960s, which was hailed as one of the three major achievements of anti-tumor drugs in the 1990s. Paclitaxel is widely applied to the treatment of cancers such as ovarian cancer, breast cancer, rectal cancer, non-small cell lung cancer, or Kaposi's sarcoma (Anil K. Singla, et al. (2002) Int. J. Pharm. 235: 179-192). The structural formula of paclitaxel is shown in formula III below.

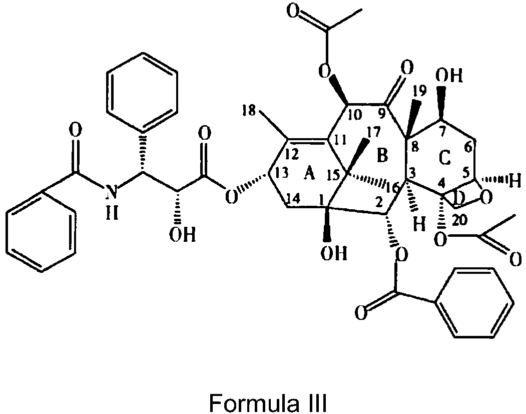

Albumin-bound paclitaxel is a novel chemotherapeutic drug formed by binding paclitaxel to albumin. Compared with conventional solvent-based paclitaxel, albumin-bound paclitaxel has better efficacy and good solubility, and is more convenient for clinical application. Albumin-bound paclitaxel can increase the concentration of drugs outside the tumor through the albumin receptor (Gp60) transcytosis pathway and the tumor extracellular matrix-bound secreted protein acidic and rich in cysteine (SPARC) pathway, thereby increasing the anti-tumor activity. Studies have reported that the binding between paclitaxel and albumin is dominated by electrostatic interaction and hydrophobic interaction (Chen Dongxiao, Zhang Juanmei, Li Jianye et al., Research Status and Progress of Paclitaxel Albumin Nanoparticles [J]. Journal of Henan University (Medical Science), 2017, 36(04): 296-300. DOI:10.15991/j.cnki.41-1361/r.2017.04.020.).

Due to the limited efficacy of single-drug chemotherapy, and the toxic and side effects and drug resistance caused by large doses, the diseases of many tumor patients still cannot be controlled for a long time after the treatment with chemotherapeutic drugs. Therefore, the development of more effective treatment means or antibody and chemotherapeutic drug combination treatment regimens is of great clinical significance for reducing the generation of drug resistance, improving the *in vivo* pharmacokinetic effect of drugs, improving the therapeutic effect of drugs, and/or reducing the side effects of drugs.

### SUMMARY

Through intensive studies and creative efforts, the inventors have creatively combined the anti-CTLA4-anti-PD-1 antibody with gemcitabine and albumin-bound paclitaxel properly, and the obtained therapeutic combination has a good anti-tumor effect.

The present disclosure is detailed below:
One aspect of the present disclosure relates to a therapeutic combination comprising an anti-CTLA4-anti-PD-1 bispecific antibody and gemcitabine or a pharmaceutically acceptable salt thereof;
preferably, the therapeutic combination further comprises paclitaxel.

In some embodiments of the present disclosure, the therapeutic combination is provided, wherein the amount of the anti-CTLA4-anti-PD-1 bispecific antibody, gemcitabine or the pharmaceutically acceptable salt thereof, and/or paclitaxel is an effective amount for treating or preventing indications of the present disclosure.

In some embodiments of the present disclosure, the therapeutic combination consists of the anti-CTLA4-anti-PD-1 bispecific antibody and gemcitabine or the pharmaceutically acceptable salt thereof.

In some embodiments of the present disclosure, the therapeutic combination consists of the anti-CTLA4-anti-PD-1 bispecific antibody, gemcitabine or the pharmaceutically acceptable salt thereof, and one or more pharmaceutically acceptable auxiliary materials. In some embodiments of the present disclosure, the therapeutic combination consists of the anti-CTLA4-anti-PD-1 bispecific antibody, gemcitabine or the pharmaceutically acceptable salt thereof, and paclitaxel.

In some embodiments of the present disclosure, the therapeutic combination consists of the anti-CTLA4-anti-PD-1 bispecific antibody, gemcitabine or the pharmaceutically acceptable salt thereof, paclitaxel, and one or more pharmaceutically acceptable auxiliary materials.

In some embodiments of the present disclosure, the therapeutic combination is provided, wherein the pharmaceutically acceptable salt of gemcitabine is gemcitabine hydrochloride.

In some embodiments of the present disclosure, the therapeutic combination is provided, wherein paclitaxel is albumin-bound paclitaxel.

In some embodiments of the present disclosure, the therapeutic combination consists of the anti-CTLA4-anti-PD-1 bispecific antibody, gemcitabine or the pharmaceutically acceptable salt thereof, and albumin-bound paclitaxel.

In some embodiments of the present disclosure, the therapeutic combination consists of the anti-CTLA4-anti-PD-1 bispecific antibody, gemcitabine or the pharmaceutically acceptable salt thereof, albumin-bound paclitaxel, and one or more pharmaceutically acceptable auxiliary materials.

In some embodiments of the present disclosure, albumin-bound paclitaxel is provided, wherein paclitaxel and albumin are in a mass ratio of (1:15) to (5:1), (1:15) to (3:1), (1:15) to (2:1), (1:10) to (3:1), (1:10) to (2:1), 1:(5-15), 1:(8-12), 1:8, 1:9, 1:10, 1:11, or 1:12.

In some embodiments of the present disclosure, the therapeutic combination is provided, wherein the anti-CTLA4-anti-PD-1 bispecific antibody is in a unit dose of 100 mg-1000 mg, 200 mg-800 mg, 200 mg-500 mg, 300 mg-600 mg, 400 mg-500 mg, or 450 mg.

In some embodiments of the present disclosure, the therapeutic combination is provided, wherein the anti-CTLA4-anti-PD-1 bispecific antibody is administered at a unit dose of 0.1-100 mg, preferably 1-15 mg, 1-12 mg, 1-10 mg, or 6-10 mg, per kg body weight.

In some embodiments of the present disclosure, the therapeutic combination is provided, wherein gemcitabine or the pharmaceutically acceptable salt thereof is in a unit dose of 100-2000 mg, 500-1500 mg, 600-1300 mg, 800-1200 mg, 800-1000 mg, 800 mg, 900 mg, 1000 mg, 1100 mg, 1200 mg, or 1500 mg.

In some embodiments of the present disclosure, the therapeutic combination is provided, wherein, based on the mass (mg) of gemcitabine or the pharmaceutically acceptable salt thereof/body surface area (m²) of a subject, gemcitabine or the pharmaceutically acceptable salt thereof is administered at a unit dose of 500-1500 mg/m², 600-1300 mg/m², 800-1250 mg/m², 800-1000 mg/m², 800 mg/m², 850 mg/m², 900 mg/m², 950 mg/m², 1000 mg/m², 1050 mg/m², 1100 mg/m², 1150 mg/m², 1200 mg/m², or 1250 mg/m².

In some embodiments of the present disclosure, the therapeutic combination is provided, wherein albumin-bound paclitaxel is in a unit dose of 200-2000 mg, 500-1500 mg, 800-1200 mg, 900-1100 mg, 800 mg, 900 mg, 1000 mg, 1100 mg, or 1200 mg (based on the mass of albumin-bound paclitaxel).

In some embodiments of the present disclosure, the therapeutic combination is provided, wherein paclitaxel is in a unit dose of 20-300 mg, 50-250 mg, 80-200 mg, 100-180 mg, 120-180 mg, 125-175 mg, 135-175 mg, 125 mg, 130 mg, 135 mg, 140 mg, 145 mg, 150 mg, 155 mg, 160 mg, 165 mg, 170 mg, or 175 mg (based on the mass of paclitaxel).

In some embodiments of the present disclosure, the therapeutic combination is provided, wherein, based on the mass (mg) of albumin-bound paclitaxel/body surface area (m²) of a subject, albumin-bound paclitaxel is administered at a unit dose of 80-300 mg/m², 100-280 mg/m², 120-260 mg/m², 120-200 mg/m², 120-180 mg/m², 125-175 mg/m², 125 mg/m², 130 mg/m², 135 mg/m², 140 mg/m², 145 mg/m², 150 mg/m², 155 mg/m², 160 mg/m², 165 mg/m², 170 mg/m², 175 mg/m², or 260 mg/m².

In some embodiments of the present disclosure, the therapeutic combination is provided, wherein, based on the mass (mg) of paclitaxel/body surface area (m²) of a subject, paclitaxel is administered at a unit dose of 20-300 mg/m², 50-250 mg/m², 80-200 mg/m², 100-180 mg/m², 120-180 mg/m², 125-175 mg/m², 135-175 mg/m², 125 mg/m², 130 mg/m², 135 mg/m², 140 mg/m², 145 mg/m², 150 mg/m², 155 mg/m², 160 mg/m², 165 mg/m², 170 mg/m², or 175 mg/m².

In some embodiments of the present disclosure, the therapeutic combination is provided, wherein
the therapeutic combination is a fixed combination, e.g., a pharmaceutical composition;
the therapeutic combination is a non-fixed combination, e.g., the anti-CTLA4-anti-PD-1 bispecific antibody, gemcitabine or the pharmaceutically acceptable salt thereof, and paclitaxel in the non-fixed combination are each present in a separate pharmaceutical composition; or
gemcitabine or the pharmaceutically acceptable salt thereof and paclitaxel are in a fixed combination, e.g., in a pharmaceutical composition; the anti-CTLA4-anti-PD-1 bispecific antibody is present in a separate pharmaceutical composition.

In some embodiments of the present disclosure, the therapeutic combination is provided, wherein the pharmaceutical composition is independently a solid pharmaceutical composition or a liquid pharmaceutical composition.

In some embodiments of the present disclosure, the therapeutic combination is provided, wherein the pharmaceutical composition further comprises one or more pharmaceutically acceptable auxiliary materials, e.g., a carrier and/or an excipient.

In some embodiments of the present disclosure, the therapeutic combination is provided, wherein the pharmaceutical composition does not comprise an active pharmaceutical ingredient other than the anti-CTLA4-anti-PD-1 bispecific antibody, gemcitabine or the pharmaceutically acceptable salt thereof, and paclitaxel.

In some embodiments of the present disclosure, the therapeutic combination is provided, wherein the anti-CTLA4-anti-PD-1 bispecific antibody comprises:
a first protein functional region targeting PD-1 and
a second protein functional region targeting CTLA4,
wherein:
   the first protein functional region is an immunoglobulin, and the second protein functional region is a single chain fragment variable, wherein a heavy chain variable region of the immunoglobulin comprises HCDR1-HCDR3 having amino acid sequences set forth in SEQ ID NOs: 27-29, respectively, and a light chain variable region of the immunoglobulin comprises LCDR1-LCDR3 having amino acid sequences set forth in SEQ ID NOs: 30-32, respectively; a heavy chain variable region of the single chain fragment variable comprises HCDR1-HCDR3 having amino acid sequences set forth in SEQ ID NOs: 33-35, respectively, and a light chain variable region of the single chain fragment variable comprises LCDR1-LCDR3 having amino acid sequences set forth in SEQ ID NOs: 36-38, respectively;
      or
   the first protein functional region is a single chain fragment variable, and the second protein functional region is an immunoglobulin, wherein a heavy chain variable region of the single chain fragment variable comprises HCDR1-HCDR3 having amino acid sequences set forth in SEQ ID NOs: 27-29, respectively, and a light chain variable region of the single chain fragment variable comprises LCDR1-LCDR3 having amino acid sequences set forth in SEQ ID NOs: 30-32, respectively; a heavy chain variable region of the immunoglobulin comprises HCDR1-HCDR3 having amino acid sequences set forth in SEQ ID NOs: 33-35, respectively, and a light chain variable region of the immunoglobulin comprises LCDR1-LCDR3 having amino acid sequences set forth in SEQ ID NOs: 36-38, respectively;
   preferably, the immunoglobulin is of human IgG1 subtype, and according to the EU numbering system, the heavy chain constant region of the immunoglobulin has mutations at any 2 or 3 of positions 234, 235 and 237, and the affinity constant of the bispecific antibody to FcyRIIIa and/or C1q is reduced after the mutation as compared to that before the mutation; preferably, the affinity constant is measured by a Fortebio Octet molecular interaction instrument.

In one or more embodiments of the present disclosure, the therapeutic combination is provided, wherein the anti-CTLA4-anti-PD-1 bispecific antibody is provided, wherein after the mutation described above, the affinity constant of the bispecific antibody to FcγRIIIa, FcγRI, FcγRIIa_H131, FcγRIIIa_V158 and/or FcγRIIb is reduced as compared to that before the mutation; preferably, the affinity constant is measured by a Fortebio Octet molecular interaction instrument.

In some embodiments of the present disclosure, the therapeutic combination is provided, wherein the anti-CTLA4-anti-PD-1 bispecific antibody is provided, wherein according to the EU numbering system, the heavy chain constant region of the immunoglobulin comprises the following mutations:
L234A and L235A; or
L234A and G237A; or
L235A and G237A;
   or
L234A, L235A, and G237A.

In the present disclosure, letters before the position number represent amino acids before mutation, and letters after the position number represent amino acids after mutation, unless otherwise specified.

In some embodiments of the present disclosure, the therapeutic combination is provided, wherein the anti-CTLA4-anti-PD-1 bispecific antibody comprises:
a first protein functional region targeting PD-1 and
a second protein functional region targeting CTLA4,
wherein:
   the first protein functional region is an immunoglobulin, and the second protein functional region is a single chain fragment variable, wherein a heavy chain variable region of the immunoglobulin comprises HCDR1-HCDR3 having amino acid sequences set forth in SEQ ID NOs: 27-29, respectively, and a light chain variable region of the immunoglobulin comprises LCDR1-LCDR3 having amino acid sequences set forth in SEQ ID NOs: 30-32, respectively; a heavy chain variable region of the single chain fragment variable comprises HCDR1-HCDR3 having amino acid sequences set forth in SEQ ID NOs: 33-35, respectively, and a light chain variable region of the single chain fragment variable comprises LCDR1-LCDR3 having amino acid sequences set forth in SEQ ID NOs: 36-38, respectively;
      or
   the first protein functional region is a single chain fragment variable, and the second protein functional region is an immunoglobulin, wherein a heavy chain variable region of the single chain fragment variable comprises HCDR1-HCDR3 having amino acid sequences set forth in SEQ ID NOs: 27-29, respectively, and a light chain variable region of the single chain fragment variable comprises LCDR1-LCDR3 having amino acid sequences set forth in SEQ ID NOs: 30-32, respectively; a heavy chain variable region of the immunoglobulin comprises HCDR1-HCDR3 having amino acid sequences set forth in SEQ ID NOs: 33-35, respectively, and a light chain variable region of the immunoglobulin comprises LCDR1-LCDR3 having amino acid sequences set forth in SEQ ID NOs: 36-38, respectively;
   preferably, the immunoglobulin is of human IgG1 subtype, and according to the EU numbering system, a heavy chain constant region of the immunoglobulin comprises one of the following combinations of mutations:
      L234A and L235A; or
      L234A and G237A; or
      L235A and G237A; or
      L234A, L235A, and G237A.

In some embodiments of the present disclosure, the therapeutic combination is provided, wherein the anti-CTLA4-anti-PD-1 bispecific antibody is provided, wherein according to the EU numbering system, the heavy chain constant region of the immunoglobulin further comprises one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In one or more embodiments of the present disclosure, the therapeutic combination is provided, wherein the anti-CTLA4-anti-PD-1 bispecific antibody is in the form of IgG-scFv, i.e., the Morrison format.

In some embodiments of the present disclosure, the therapeutic combination is provided, wherein the anti-CTLA4-anti-PD-1 bispecific antibody is provided, wherein
an amino acid sequence of the heavy chain variable region of the immunoglobulin is selected from SEQ ID NO: 14 and SEQ ID NO: 18, and an amino acid sequence of the light chain variable region of the immunoglobulin is selected from SEQ ID NO: 16 and SEQ ID NO: 20; an amino acid sequence of the heavy chain variable region of the single chain fragment variable is selected from SEQ ID NO: 2, SEQ ID NO: 6, SEQ ID NO: 10, SEQ ID NO: 41 and SEQ ID NO: 43, and an amino acid sequence of the light chain variable region of the single chain fragment variable is selected from SEQ ID NO: 4, SEQ ID NO: 8, SEQ ID NO: 12, SEQ ID NO: 42 and SEQ ID NO: 44;
   or
an amino acid sequence of the heavy chain variable region of the immunoglobulin is selected from SEQ ID NO: 2, SEQ ID NO: 6, SEQ ID NO: 10, SEQ ID NO: 41 and SEQ ID NO: 43, and an amino acid sequence of the light chain variable region of the immunoglobulin is selected from SEQ ID NO: 4, SEQ ID NO: 8, SEQ ID NO: 12, SEQ ID NO: 42 and SEQ ID NO: 44; an amino acid sequence of the heavy chain variable region of the single chain fragment variable is selected from SEQ ID NO: 14 and SEQ ID NO: 18, and an amino acid sequence of the light chain variable region of the single chain fragment variable is selected from SEQ ID NO: 16 and SEQ ID NO: 20.

In some embodiments of the present disclosure, the therapeutic combination is provided, wherein the anti-CTLA4-anti-PD-1 bispecific antibody is selected from any one of the following (1)-(20):
(1)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(2)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(3)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(4)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(5)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(6)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(7)
   an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(8)
   an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(9)
   an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(10)
   an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(11)
   an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(12)
   an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(13)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(14)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(15)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(16)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(17)
   an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(18)
   an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(19)
   an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
   and
(20)
   an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 18, and an amino acid sequence of the light chain variable region of the single chain fragment variable is set forth in SEQ ID NO: 20.

In some embodiments of the present disclosure, the therapeutic combination is provided, wherein the anti-CTLA4-anti-PD-1 bispecific antibody is provided:
an amino acid sequence of the heavy chain of the immunoglobulin is set forth in SEQ ID NO: 40, and an amino acid sequence of the light chain is set forth in SEQ ID NO: 24.

In some embodiments of the present disclosure, the therapeutic combination is provided, wherein the anti-CTLA4-anti-PD-1 bispecific antibody is provided, wherein the first protein functional region is linked, either directly or via a linker fragment, to the second protein functional region, and/or the heavy chain variable region of the single chain fragment variable is linked, either directly or via a linker fragment, to the light chain variable region of the single chain fragment variable.

In some embodiments of the present disclosure, the therapeutic combination is provided, wherein the anti-CTLA4-anti-PD-1 bispecific antibody is provided, wherein the linker fragment is a polypeptide set forth in SEQ ID NO: 45 (GGGGS), or a polypeptide consisting of a plurality (e.g., 2, 3, 4, 5, or 6) of polypeptides set forth in SEQ ID NO: 45 linked in series.

In some embodiments of the present disclosure, the therapeutic combination is provided, wherein the anti-CTLA4-anti-PD-1 bispecific antibody is provided, wherein the numbers of the first protein functional region and the second protein functional region are each independently 1, 2, or more.

In one or more embodiments of the present disclosure, the therapeutic combination is provided, wherein the anti-CTLA4-anti-PD-1 bispecific antibody is provided, wherein the first protein functional region is 1 in number, and the second protein functional region is 2 in number.

In one or more embodiments of the present disclosure, the therapeutic combination is provided, wherein the anti-CTLA4-anti-PD-1 bispecific antibody is provided, wherein the single chain fragment variable is linked to the C-terminus of the heavy chain of the immunoglobulin. Since an immunoglobulin has two heavy chains, two single chain fragment variable molecules are linked to one immunoglobulin molecule. Preferably, the two single chain fragment variable molecules are identical. Preferably, the single chain fragment variable is linked to the C-terminus of the heavy chain of the immunoglobulin by forming an amide bond via the aforementioned linker fragment.

In one or more embodiments of the present disclosure, the constant regions of the immunoglobulin are humanized. For example, the heavy chain constant region is Ig gamma-1 chain C region, ACCESSION: P01857, and the light chain constant region is Ig kappa chain C region, ACCESSION: P01834.

In one or more embodiments of the present disclosure, the therapeutic combination is provided, wherein the anti-CTLA4-anti-PD-1 bispecific antibody binds to CTLA4 protein and/or PD-1 protein with a K_{D} less than about 10⁻⁵ M, e.g., less than about 10⁻⁶ M, 10⁻⁷ M, 10⁻⁸ M, 10⁻⁹ M, or 10⁻¹⁰ M or less.

In one or more embodiments of the present disclosure, the therapeutic combination is provided, wherein the anti-CTLA4-anti-PD-1 bispecific antibody is a monoclonal antibody. In one or more embodiments of the present disclosure, the therapeutic combination is provided, wherein the anti-CTLA4-anti-PD-1 bispecific antibody is a humanized antibody. In some embodiments of the present disclosure, the therapeutic combination is provided, wherein the anti-CTLA4-anti-PD-1 bispecific antibody is provided, wherein the single chain fragment variables are linked to the C-termini of two heavy chains of the immunoglobulin, respectively.

In some embodiments of the present disclosure, the therapeutic combination is provided, wherein the anti-CTLA4-anti-PD-1 bispecific antibody comprises:
a first protein functional region targeting PD-1 and
a second protein functional region targeting CTLA4,
the first protein functional region is 1 in number, and the second protein functional region is 2 in number;
wherein the first protein functional region is an immunoglobulin, and the second protein functional region is a single chain fragment variable;
an amino acid sequence of the heavy chain of the immunoglobulin is set forth in SEQ ID NO: 40, and an amino acid sequence of the light chain is set forth in SEQ ID NO: 24;
an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
the single chain fragment variable is linked to the C-terminus of the heavy chain of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NO: 25 and SEQ ID NO: 26;
preferably, amino acid sequences of the first linker fragment and the second linker fragment are set forth in SEQ ID NO: 26.

Another aspect of the present disclosure relates to a kit product comprising the therapeutic combination according to any embodiment of the present disclosure, and a package insert.

In some embodiments of the present disclosure, the kit product comprises a first product, a second product and a third product in separate packages,
wherein,
the first product comprises the anti-CTLA4-anti-PD-1 bispecific antibody according to any embodiment of the present disclosure;
the second product comprises gemcitabine or the pharmaceutically acceptable salt thereof;
the third product comprises albumin-bound paclitaxel;
preferably, the first product, the second product and the third product further independently comprise one or more pharmaceutically acceptable auxiliary materials;
preferably, the kit product further comprises a package insert.

In one or more embodiments of the present disclosure, the kit product is provided, wherein the unit dose of the first product is 100 mg-1000 mg, 200 mg-800 mg, 200 mg-500 mg, 300 mg-600 mg, 400 mg-500 mg, or 450 mg, based on the mass of the anti-CTLA4-anti-PD-1 bispecific antibody.

Yet another aspect of the present disclosure relates to use of the therapeutic combination according to any embodiment of the present disclosure in preparing a medicament for treating or preventing a tumor;
preferably, the tumor is selected from one or more of pancreatic cancer, melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the pancreatic cancer is selected from pancreatic ductal adenocarcinoma and pancreatic adenosquamous carcinoma;
preferably, the pancreatic cancer is advanced or metastatic pancreatic cancer;
preferably, the lung cancer is selected from one or more of non-small cell lung cancer, small cell lung cancer and squamous cell lung cancer;
preferably, the non-small cell lung cancer is intermediate or advanced non-small cell lung cancer;
preferably, the gastric cancer is gastric adenocarcinoma or gastroesophageal junction adenocarcinoma.

The therapeutic combination according to any embodiment of the present disclosure for use in the treatment or prevention of a tumor is provided, wherein
preferably, the tumor is selected from one or more of pancreatic cancer, melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the pancreatic cancer is selected from pancreatic ductal adenocarcinoma and pancreatic adenosquamous carcinoma;
preferably, the pancreatic cancer is advanced or metastatic pancreatic cancer;
preferably, the lung cancer is selected from one or more of non-small cell lung cancer, small cell lung cancer and squamous cell lung cancer;
preferably, the non-small cell lung cancer is intermediate or advanced non-small cell lung cancer;
preferably, the gastric cancer is gastric adenocarcinoma or gastroesophageal junction adenocarcinoma.

Yet another aspect of the present disclosure relates to a method for treating or preventing a tumor, comprising a step of administering to a subject in need an effective amount of the therapeutic combination according to any embodiment of the present disclosure;
preferably, the tumor is selected from one or more of pancreatic cancer, melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the pancreatic cancer is selected from pancreatic ductal adenocarcinoma and pancreatic adenosquamous carcinoma;
preferably, the pancreatic cancer is advanced or metastatic pancreatic cancer;
preferably, the lung cancer is selected from one or more of non-small cell lung cancer, small cell lung cancer and squamous cell lung cancer;
preferably, the non-small cell lung cancer is intermediate or advanced non-small cell lung cancer;
preferably, the gastric cancer is gastric adenocarcinoma or gastroesophageal junction adenocarcinoma.

In one or more embodiments of the present disclosure, the method for treating or preventing a tumor is provided, wherein the step of administering to a subject in need an effective amount of the anti-CTLA4-anti-PD-1 bispecific antibody is before or after a surgical treatment and/or before or after a radiotherapy.

In one or more embodiments of the present disclosure, the method for treating or preventing a tumor is provided, wherein
the anti-CTLA4-anti-PD-1 bispecific antibody is administered at a unit dose of 0.1-100 mg, preferably 1-15 mg, 1-12 mg, 1-10 mg (e.g., 1 mg, 2 mg, 3 mg, 4 mg, 5 mg, 6 mg, 7 mg, 8 mg, 9 mg, or 10 mg), or 6-10 mg, per kg body weight; alternatively, the anti-CTLA4-anti-PD-1 bispecific antibody is administered at a unit dose of 10-1000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, or about 1000 mg), preferably 50-500 mg, 100-400 mg, 150-300 mg, 150-250 mg, or 200 mg in each subject;
preferably, administration is performed once every 3 days, 4 days, 5 days, 6 days, 10 days, 1 week, 2 weeks, or 3 weeks;
preferably, the route of administration is intravenous drip infusion or intravenous injection. In some embodiments, the administration of the anti-CTLA4-anti-PD-1 bispecific antibody is performed in cycles of 2 weeks (14 days) or 3 weeks (21 days), and preferably, the anti-CTLA4-anti-PD-1 bispecific antibody is administered intravenously on the first day (D1) of each cycle. For example, the anti-CTLA4-anti-PD-1 bispecific antibody is administered once every two weeks (q2w) or three weeks (q3w).

Based on the mass (mg) of gemcitabine or the pharmaceutically acceptable salt thereof/body surface area (m²) of a subject, gemcitabine or the pharmaceutically acceptable salt thereof is administered at a unit dose of 500-1500 mg/m², 600-1300 mg/m², 800-1250 mg/m², 800-1000 mg/m², 800 mg/m², 850 mg/m², 900 mg/m², 950 mg/m², 1000 mg/m², 1050 mg/m², 1100 mg/m², 1150 mg/m², 1200 mg/m², or 1250 mg/m²;
preferably, administration is performed once every 3 days, 4 days, 5 days, 6 days, 10 days, 1 week, or 2 weeks;
preferably, the route of administration is intravenous drip infusion or intravenous injection. In some embodiments, gemcitabine or the pharmaceutically acceptable salt thereof is administered once a week for three consecutive weeks, followed by a one-week interruption as a course of treatment; alternatively, gemcitabine or the pharmaceutically acceptable salt thereof is administered once a week for two consecutive weeks, followed by a one-week interruption as a course of treatment. Preferably, treatment is performed for 2-4 consecutive courses.

Based on the mass (mg) of albumin-bound paclitaxel/body surface area (m²) of a subject, albumin-bound paclitaxel is administered at a unit dose of 80-300 mg/m², 100-280 mg/m², 120-260 mg/m², 120-200 mg/m², 120-180 mg/m², 125-175 mg/m², 125 mg/m², 130 mg/m², 135 mg/m², 140 mg/m², 145 mg/m², 150 mg/m², 155 mg/m², 160 mg/m², 165 mg/m², 170 mg/m², 175 mg/m², or 260 mg/m².

Based on the mass (mg) of paclitaxel/body surface area (m²) of a subject, paclitaxel is administered at a unit dose of 20-300 mg/m², 50-250 mg/m², 80-200 mg/m², 100-180 mg/m², 120-180 mg/m², 125-175 mg/m², 135-175 mg/m², 125 mg/m², 130 mg/m², 135 mg/m², 140 mg/m², 145 mg/m², 150 mg/m², 155 mg/m², 160 mg/m², 165 mg/m², 170 mg/m², or 175 mg/m².

Preferably, administration is performed once every 3 days, 4 days, 5 days, 6 days, 10 days, 1 week, 2 weeks, 3 weeks, or 4 weeks;
preferably, the route of administration is intravenous drip infusion or intravenous injection. In one or more embodiments of the present disclosure, the method for treating or preventing a tumor is provided, wherein the anti-CTLA4-anti-PD-1 bispecific antibody, gemcitabine, and paclitaxel (e.g., albumin-bound paclitaxel) are administered simultaneously or sequentially.

In one or more embodiments of the present disclosure, the method for treating or preventing a tumor comprises a first stage and a second stage, wherein the anti-CTLA4-anti-PD-1 bispecific antibody, gemcitabine or the pharmaceutically acceptable salt thereof, and paclitaxel (e.g., albumin-bound paclitaxel) are administered in the first stage.

In one or more embodiments of the present disclosure, the method for treating or preventing a tumor is provided, wherein the anti-CTLA4-anti-PD-1 bispecific antibody and gemcitabine or the pharmaceutically acceptable salt thereof are administered in the second stage.

In one or more embodiments of the present disclosure, the method for treating or preventing a tumor is provided, wherein the anti-CTLA4-anti-PD-1 bispecific antibody is administered in the second stage of the treatment.

The first stage is a combination therapy stage, comprising 1-10 courses of treatment, 2-8 courses of treatment, or 4-6 courses of treatment, each course of treatment lasting for 2-4 weeks, preferably 4 weeks.

The second stage is a maintenance treatment stage.

In the first stage and the second stage, the dosage, frequency of administration, and the like of each pharmaceutical ingredient may be made with reference to the foregoing description or the pharmaceutical instructions, or according to the advice of a physician.

Antibody drugs, especially monoclonal antibodies (mAbs), have achieved good efficacy in the treatment of various diseases. Conventional methods for acquiring these therapeutic antibodies include immunizing animals with an antigen and acquiring antibodies targeting the antigen in the immunized animals, or modifying those antibodies with lower affinity for the antigen by affinity maturation.

The variable regions of the light chain and the heavy chain determine the binding of the antigen; the variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3; defined by Kabat et al., see Sequences of Proteins of Immunological Interest, Fifth Edition (1991), Volumes 1-3, NIH Publication 91-3242, Bethesda Md).

The amino acid sequences of the CDR regions of the monoclonal antibody sequences in (1)-(9) below are analyzed by technical means well known to those skilled in the art, for example, by a VBASE2 database, and the results are as follows:

### (1) 14C12

An amino acid sequence of the heavy chain variable region is set forth in SEQ ID NO: 14, and an amino acid sequence of the light chain variable region is set forth in SEQ ID NO: 16.

Amino acid sequences of the 3 CDR regions of the heavy chain variable region are as follows:
HCDR1: GFAFSSYD (SEQ ID NO: 27)
HCDR2: ISGGGRYT (SEQ ID NO: 28)
HCDR3: ANRYGEAWFAY (SEQ ID NO: 29)

Amino acid sequences of the 3 CDR regions of the light chain variable region are as follows:
LCDR1: QDINTY (SEQ ID NO: 30)
LCDR2: RAN (SEQ ID NO: 31)
LCDR3: LQYDEFPLT (SEQ ID NO: 32)

### (2) 14C12H1L1

An amino acid sequence of the heavy chain variable region is set forth in SEQ ID NO: 18, and an amino acid sequence of the light chain variable region is set forth in SEQ ID NO: 20.

The amino acid sequences of the 3 CDR regions of the heavy chain variable region are identical to those of 14C12.

The amino acid sequences of the 3 CDR regions of the light chain variable region are identical to those of 14C12.

### (3) 4G10

An amino acid sequence of the heavy chain variable region is set forth in SEQ ID NO: 2, and an amino acid sequence of the light chain variable region is set forth in SEQ ID NO: 4.

Amino acid sequences of the 3 CDR regions of the heavy chain variable region are as follows:
HCDR1: GYSFTGYT (SEQ ID NO: 33)
HCDR2: INPYNNIT (SEQ ID NO: 34)
HCDR3: ARLDYRSY (SEQ ID NO: 35)

Amino acid sequences of the 3 CDR regions of the light chain variable region are as follows:
LCDR1: TGAVTTSNF (SEQ ID NO: 36)
LCDR2: GTN (SEQ ID NO: 37)
LCDR3: ALWYSNHWV (SEQ ID NO: 38)

### (4) 4G10H1L1

An amino acid sequence of the heavy chain variable region is set forth in SEQ ID NO: 6, and an amino acid sequence of the light chain variable region is set forth in SEQ ID NO: 8.

The amino acid sequences of the 3 CDR regions of the heavy chain variable region are identical to those of 4G10.

The amino acid sequences of the 3 CDR regions of the light chain variable region are identical to those of 4G10.

### (5) 4G10H3L3

An amino acid sequence of the heavy chain variable region is set forth in SEQ ID NO: 10, and an amino acid sequence of the light chain variable region is set forth in SEQ ID NO: 12.

The amino acid sequences of the 3 CDR regions of the heavy chain variable region are identical to those of 4G10.

The amino acid sequences of the 3 CDR regions of the light chain variable region are identical to those of 4G10.

### (6) BiAb001(M)

Amino acid sequences of the 9 CDR regions associated with the heavy chain variable region are as follows:
HCDR1: GFAFSSYD (SEQ ID NO: 27)
HCDR2: ISGGGRYT (SEQ ID NO: 28)
HCDR3: ANRYGEAWFAY (SEQ ID NO: 29)
HCDR4: GYSFTGYT (SEQ ID NO: 33)
HCDR5: INPYNNIT (SEQ ID NO: 34)
HCDR6: ARLDYRSY (SEQ ID NO: 35)
HCDR7: TGAVTTSNF (SEQ ID NO: 36)
HCDR8: GTN (SEQ ID NO: 37)
HCDR9: ALWYSNHWV (SEQ ID NO: 38)

Amino acid sequences of the 3 CDR regions associated with the light chain variable region are as follows:
LCDR1: QDINTY (SEQ ID NO: 30)
LCDR2: RAN (SEQ ID NO: 31)
LCDR3: LQYDEFPLT (SEQ ID NO: 32)

### (7) BiAb002(M)

The amino acid sequences of the 9 CDR regions associated with the heavy chain variable region are identical to those of BiAb001(M).

The amino acid sequences of the 3 CDR regions associated with the light chain variable region are identical to those of BiAb001(M).

### (8) BiAb003(M)

The amino acid sequences of the 9 CDR regions associated with the heavy chain variable region are identical to those of BiAb001(M).

The amino acid sequences of the 3 CDR regions associated with the light chain variable region are identical to those of BiAb001(M).

### (9) BiAb004(M)

The amino acid sequences of the 9 CDR regions associated with the heavy chain variable region are identical to those of BiAb001(M).

The amino acid sequences of the 3 CDR regions associated with the light chain variable region are identical to those of BiAb001(M).

For the antibody BiAb004(hG1TM) of the present disclosure, amino acid mutations are introduced into the non-variable region of BiAb004(M). According to the EU numbering system, amino acid mutations are introduced at positions 234, 235 and 237:
BiAb004(hG1TM) is obtained by introducing a leucine-to-alanine point mutation at position 234 (L234A), a leucine-to-alanine point mutation at position 235 (L235A), and a glycine-to-alanine point mutation at position 237 (G237A) in the hinge region of the heavy chain.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below.

As used herein, when referring to the amino acid sequence of CTLA4 protein (cytotoxic T-lymphocyte-associated antigen 4), it includes but is not limited to the full length of CTLA4 protein, or the extracellular fragment CTLA4 ECD of CTLA4 or a fragment comprising CTLA4 ECD; also included are fusion proteins of CTLA4 ECD, such as a fragment fused to an Fc protein fragment of a mouse or human IgG (mFc or hFc). However, those skilled in the art will appreciate that in the amino acid sequence of the CTLA4 protein, mutations or variations (including but not limited to, substitutions, deletions, and/or additions) can be naturally produced or artificially introduced without affecting biological functions thereof. Therefore, in the present disclosure, the term "CTLA4 protein" shall include all such sequences, including their natural or artificial variants. In addition, when describing a sequence fragment of the CTLA4 protein, it also includes the corresponding sequence fragments in their natural or artificial variants.

As used herein, when referring to the amino acid sequence of PD-1 protein, it includes but is not limited to the full length of PD-1 protein (NCBI GenBank: NM_005018), or the extracellular fragment PD-1 ECD of PD-1 or a fragment comprising PD-1 ECD; also included are fusion proteins of PD-1 ECD, such as a fragment fused to an Fc protein fragment of a mouse or human IgG (mFc or hFc). However, those skilled in the art will appreciate that in the amino acid sequence of the PD-1 protein, mutations or variations (including but not limited to, substitutions, deletions, and/or additions) can be naturally produced or artificially introduced without affecting biological functions thereof. Therefore, in the present disclosure, the term "PD-1 protein" shall include all such sequences, including their natural or artificial variants. In addition, when describing a sequence fragment of the PD-1 protein, it also includes the corresponding sequence fragments in their natural or artificial variants.

As used herein, unless otherwise specified, the B7 is B7-1 and/or B7-2; specific protein sequences thereof are those known in the prior art, and reference may be made to sequences disclosed in the existing literature or GenBank. For example, B7-1 (CD80, NCBI Gene ID: 941); B7-2 (CD86, NCBI Gene ID: 942).

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair consisting of one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains can be classified into κ and λ light chains. Heavy chains are generally classified into µ, δ, γ, α and ε, and the antibodies are defined as IgM, IgD, IgG, IgA and IgE isotypes, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of the classical complement system. The V_{H} and V_{L} regions can be further subdivided into hypervariable regions (called complementarity determining regions, or CDRs), and conservative regions called framework regions (FRs) are distributed between the CDRs. Each V_{H} and V_{L} consists of 3 CDRs and 4 FRs arranged from the amino-terminus to the carboxyl-terminus in the following order: FR1, CDR1, FR2, CDR2, FR3, CDR3, FR4. The variable regions (V_{H} and V_{L}) of each heavy chain/light chain pair form an antibody binding site. The assignment of amino acids to each region or domain follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883. In particular, the heavy chain may further comprise more than 3 CDRs, such as 6, 9, or 12. For example, in the bifunctional antibody of the present disclosure, the heavy chain may be an scFv with the C-terminus of the heavy chain of an IgG antibody linked to another antibody, and in this case, the heavy chain comprises 9 CDRs. The term "antibody" is not limited by any specific method for producing the antibody. For example, the antibody includes, in particular, a recombinant antibody, a monoclonal antibody and a polyclonal antibody. The antibody may be antibodies of different isotypes, such as IgG (e.g., subtype IgG1, IgG2, IgG3, or IgG4), IgA1, IgA2, IgD, IgE, or IgM.

Antigen-binding fragments (e.g., the above-mentioned antibody fragments) of antibodies can be obtained from given antibodies by using conventional techniques known to those skilled in the art (e.g., DNA recombination, or enzymatic or chemical cleavage), and the antigen-binding fragments of the antibodies are screened for specificity in the same way as for intact antibodies.

As used herein, unless otherwise clearly defined in the context, when referring to the term "antibody", it includes not only intact antibodies but also antigen-binding fragments of antibodies.

As used herein, the terms "mAb" and "monoclonal antibody" refer to an antibody or a fragment of an antibody that is derived from a group of highly homologous antibody molecules, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies that generally recognize different epitopes on an antigen. Monoclonal antibodies can generally be obtained using the hybridoma technique first reported by Kohler et al. (Kohler et al., Nature, 256:495, 1975), but can also be obtained using DNA recombination (see, e.g., U.S. Patent No. 4,816,567).

As used herein, the term "humanized antibody" refers to an antibody or an antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today, 21: 397-402 (2000).

As used herein, the term "epitope" refers to a site on the antigen that an immunoglobulin or antibody specifically binds to. The "epitope" is also referred to in the art as an "antigenic determinant". The epitope or antigenic determinant generally consists of chemically active surface groups of molecules such as amino acids, carbohydrates, or sugar side chains, and usually has specific three-dimensional structural characteristics and specific charge characteristics. For example, an epitope typically comprises at least 3, 4, 5, 6, 7, 8, 9, 10, 11, 12, 13, 14, or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to being obtained by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "E. coli expression system" refers to an expression system consisting of E. coli (strain) and a vector, wherein the E. coli (strain) is derived from a commercially available strain, such as but not limited to GI698, ER2566, BL21 (DE3), B834 (DE3), and BLR (DE3).

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *Escherichia coli* or *Bacillus subtilis,* fungal cells such as yeast cells or *Aspergillus,* insect cells such as S2 Drosophila cells or Sf9, or animal cells such as fibroblasts, CHO cells, COS cells, NSO cells, HeLa cells, BHK cells, HEK 293 cells or human cells.

As used herein, the term "specifically bind" refers to a non-random binding reaction between two molecules, such as a reaction between an antibody and an antigen it targets. In certain embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10-¹⁰ M or less. In some embodiments of the present disclosure, the term "target" refers to specific binding.

As used herein, the term "K_{D}" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, an antibody binds to an antigen with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M or less, for example, as measured by a BIACORE surface plasmon resonance (SPR) instrument or a Fortebio Octet molecular interaction instrument.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable auxiliary material" refers to a carrier and/or an excipient that is pharmacologically and/or physiologically compatible with the subject and the active ingredient. Such auxiliary materials are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more frequently in clinical trials.

As used herein, the term "effective amount" refers to an amount sufficient to obtain or at least partially obtain a desired effect. For example, a prophylactically effective amount (e.g., for a disease associated with binding of CTLA4 to B7 or CTLA4 overactivity, such as a tumor) is an amount sufficient to prevent, stop, or delay the onset of the disease (e.g., a disease associated with binding of CTLA4 to B7 or CTLA4 overactivity, such as a tumor); a therapeutically effective amount is an amount sufficient to cure or at least partially stop a disease and its complications in a patient suffering from the disease. It is undoubtedly within the ability of those skilled in the art to determine such an effective amount. For example, the amount effective for therapeutic purposes will depend on the severity of the disease to be treated, the overall state of the patient's own immune system, the general condition of the patient such as age, body weight, and gender, the route of administration, and other treatments given concurrently, etc.

A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

In the present disclosure, the terms "first" (e.g., first protein functional region, first linker fragment, or first product) and "second" (e.g., second protein functional region, second linker fragment, or second product) are used for distinguishing or clarity in expression and do not carry typical sequential meanings, unless otherwise specified.

In the present disclosure, "about" or "approximately" means that the value or physical quantity defined fluctuates within a range of 10%, 20%, or 30%, unless otherwise specified. For example, about 100 minutes or approximately 100 minutes may be 90 minutes to 110 minutes, 80 minutes to 120 minutes, or 70 minutes to 130 minutes.

### Beneficial Effects of the Present Disclosure

The present disclosure achieves one or more of the following technical effects (1) to (6):
(1) The therapeutic combination of the present disclosure has a good effect on treating or preventing a tumor.
(2) In the therapeutic combination of the present disclosure, the anti-CTLA4-anti-PD-1 bispecific antibody has a synergistic effect with gemcitabine and/or paclitaxel (e.g., albumin-bound paclitaxel), thereby achieving a synergistic effect in treating or preventing a tumor, which is superior to the effect of using the anti-CTLA4-anti-PD-1 bispecific antibody alone, or to the effect of the combination of other anti-PD-1 monoclonal antibodies (e.g., Opdivo) + anti-CTLA4 monoclonal antibodies (e.g., Yervoy) + gemcitabine and/or paclitaxel (e.g., albumin-bound paclitaxel); wherein the tumor includes, but is not limited to: pancreatic cancer, melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, or nasopharyngeal cancer.
(3) The present disclosure may be beneficial for reducing the amount and/or effective concentration of gemcitabine.
(4) The present disclosure may be beneficial for reducing the toxic and side effects of gemcitabine.
(5) The present disclosure may be beneficial for reducing the amount and/or effective concentration of paclitaxel.
(6) The present disclosure may be beneficial for reducing the toxic and side effects of paclitaxel.

### BRIEF DESCRIPTION OF THE DRAWINGS

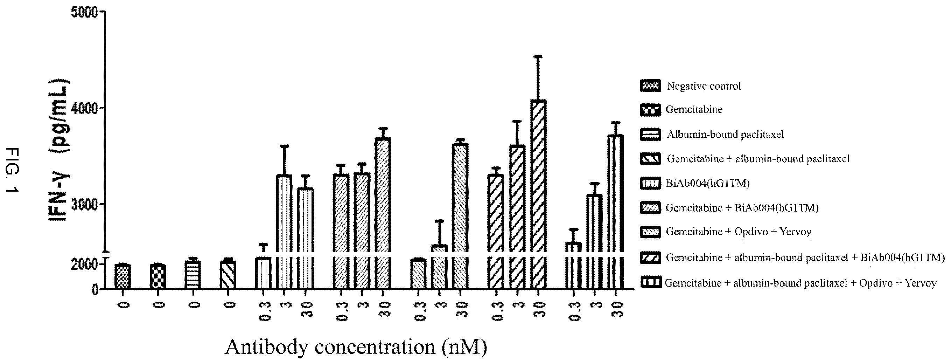

FIG. 1 shows the promotion of INF-γ secretion in a mixed lymphocyte reaction system by an anti-CTLA4-anti-PD-1 bispecific antibody in combination with gemcitabine and albumin-bound paclitaxel.

### DETAILED DESCRIPTION

The embodiments of the present disclosure will be described in detail below with reference to the examples. Those skilled in the art will appreciate that the following examples are only for illustrating the present disclosure, and should not be construed as limitations to the scope of the present disclosure. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

In the following examples of the present disclosure:
Human peripheral blood mononuclear cells were isolated and prepared in Akeso Biopharma Inc.
Raji-PDL1 is a cell expressing human PD-L1 constructed by Akeso Biopharma Inc. on the basis of the human B cell line Raji via transfection.
Ficoll-Paque^{™} PLUS (or Ficoll-Paque PLUS) was purchased from Cytiva.
RPMI 1640 medium, DMEM medium, Trypsin-EDTA (0.25%) phenol red and Blasticidin were all purchased from Gibco.
*Staphylococcus aureus* enterotoxin antigen (SEB) was purchased from Toxin Technology, Cat. No. BT202.
FBS was purchased from Excell bio.
Gemcitabine (abbreviated as Gem) was purchased from MCE, Cat. No. HY-17026.
Albumin-bound paclitaxel, also known as Abraxane (abbreviated as Abra), was purchased from MCE, Cat. No. HY-P99974.

### Preparation Example 1: Sequence Design of Anti-CTLA4 Antibodies

The amino acid sequences and encoding nucleotide sequences of the heavy and light chains of the anti-CTLA4 antibody 4G10 and its humanized antibodies 4G10H1L1 and 4G10H3L3 are identical to those of 4G10, 4G10H1L1 and 4G10H3L3 in Chinese Patent Publication No. CN106967172A, respectively.
(1) Heavy and light chain variable region sequences of 4G10
   Nucleotide sequence of the heavy chain variable region: (372 bp) 
CAGGTCAAGCTGCAGGAGTCTGGACCTGAGCTGGTGAAGCCTGGAGCTTCAATGA
AGATATCCTGCAAGGCTTCTGGTTACTCATTCACTGGCTACACCATGAACTGGGTGA
AGCAGAGCCATGGAAAGAACCTTGAATGGATTGGACTTATTAATCCTTACAATAATATT
ACTAACTACAACCAGAAGTTCATGGGCAAGGCCACATTTACTGTAGACAAGTCATCC
AGCACAGCCTACATGGAACTCCTCAGACTGACATCTGAAGACTCTGGAGTCTATTTC
TGTGCAAGACTCGACTATAGGTCTTATTGGGGCCAAGGGACTCTGGTCACTGTCTCT
GCAGCCAAAACGACACCCCCATCTGTCTAT (SEQ ID NO: 1)
   The encoded amino acid sequence: (124 aa) 
QVKLQESGPELVKPGASMKISCKASGYSFTGYTMNWVKQSHGKNLEWIGLINPYNNIT
NYNQKFMGKATFTVDKSSSTAYMELLRLTSEDSGVYFCARLDYRSYWGQGTLVTVSAA
KTTPPSVY (SEQ ID NO: 2)
   Nucleotide sequence of the light chain variable region: (378 bp) 
CAGGCTGTTGTGACTCAGGAATCTGCACTCACCACATCACCTGGTGAAACAGTCAC
ACTCACTTGTCGCTCAAGTACTGGGGCTGTTACAACTAGTAACTTTGCCAACTGGGT
CCAAGAAAAACCAGATCATTTATTCACTAGTCTAATAGGTGGTACCAACAACCGAGCT
CCAGGTGTTCCTGCCAGATTCTCAGGCTCCCTGATTGGAGACAAGGCTGCCCTCAC
CATCACAGGGGCACAGACTGAGGATGAGGCAATATATTTCTGTGCTCTATGGTACAG
CAACCATTGGGTGTTCGGTGGAGGAACCAAACTGACTGTCCTAGGCCAGCCCAAGT
CTTCGCCATCAGTCACCCTGTTTCAAGGGCAATTCTGC (SEQ ID NO: 3)
   The encoded amino acid sequence: (126 aa) 
QAVVTQESALTTSPGETVTLTCRSSTGAVTTSNFANWVQEKPDHLFTSLIGGTNNRAPG
VPARFSGSLIGDKAALTITGAQTEDEAIYFCALWYSNHWVFGGGTKLTVLGQPKSSPSV
TLFQGQFC (SEQ ID NO: 4)
(2) Heavy and light chain variable region sequences of humanized monoclonal antibody 4G10H1L1
   Nucleotide sequence of the heavy chain variable region (4G10H1V): (345 bp) 
CAGGTGCAGCTGGTGGAGTCTGGGGCCGAGCTGGTGAAGCCCGGCGCCTCCATG
AAGATCTCTTGCAAGGCCAGCGGATACAGTTTCACTGGCTATACCATGAACTGGGTC
AAACAGGCTCCAGGACAGGGACTGGAGTGGATCGGGCTGATTAATCCTTACAACAA
CATCACCAACTACAACCAGAAGTTCATGGGAAAAGCAACCTTTACAGTGGACAAGAG
CATTTCCACAGCCTACATGGAACTGAGCCGGCTGACTTCAGACGATAGCGGGGTCT
ATTTTTGTGCAAGGCTGGATTATCGCTCTTACTGGGGGCAGGGAACTCTGGTCACT
GTCTCCGCT (SEQ ID NO: 5)
   The encoded amino acid sequence: (115 aa) 
QVQLVESGAELVKPGASMKISCKASGYSFTGYTMNWVKQAPGQGLEWIGLINPYNNIT
NYNQKFMGKATFTVDKSISTAYMELSRLTSDDSGVYFCARLDYRSYWGQGTLVTVSA
(SEQ ID NO: 6)
   Nucleotide sequence of the light chain variable region (4G10L1V): (327 bp) 
CAGGCTGTCGTCACTCAGGAACCTTCACTGACTGTGAGCCCAGGAGGAACTGTCA
CCCTGACATGCGGAAGCTCCACCGGAGCAGTGACCACATCCAACTTCGCCAATTGG
GTCCAGGAAAAGCCAGGCCAGGCATTTCGATCCCTGATCGGAGGCACAAACAATCG
GGCTTCTTGGGTGCCCGCAAGATTCTCAGGAAGCCTGCTGGGGGGAAAAGCCGCT
CTGACCATTAGTGGCGCTCAGCCTGAGGACGAAGCCGAGTACTTCTGCGCTCTGTG
GTATAGCAACCACTGGGTGTTTGGCGGGGGAACAAAGCTGACTGTGCTG (SEQ ID
NO: 7)
   The encoded amino acid sequence: (109 aa) 
QAVVTQEPSLTVSPGGTVTLTCGSSTGAVTTSNFANWVQEKPGQAFRSLIGGTNNRAS
WVPARFSGSLLGGKAALTISGAQPEDEAEYFCALWYSNHWVFGGGTKLTVL (SEQ ID
NO: 8)
(3) Heavy and light chain variable region sequences of humanized monoclonal antibody 4G10H3L3
   Nucleotide sequence of the heavy chain variable region (4G10H3V): (345 bp) 
CAGGTGCAGCTGGTCGAGTCTGGGGCCGAAGTGAAGAAACCCGGCGCCTCAGTG
AAGGTCAGCTGCAAGGCCAGCGGGTACAGTTTCACTGGATATACCATGAACTGGGT
CCGACAGGCCCCTGGCCAGGGGCTGGAGTGGATCGGCCTGATTAACCCTTACAAC
AACATCACTAACTACGCACAGAAGTTCCAGGGGAGAGTGACCTTTACAGTGGACAC
CAGCATTTCCACAGCCTACATGGAACTGTCCCGGCTGAGATCTGACGATACAGGCG
TGTACTTCTGCGCTAGGCTGGATTACCGCAGCTATTGGGGACAGGGCACACTGGTG
ACTGTCAGCGCA (SEQ ID NO: 9)
   The encoded amino acid sequence (4G10H3V): (115 aa) 
QVQLVESGAEVKKPGASVKVSCKASGYSFTGYTMNWVRQAPGQGLEWIGLINPYNNIT
NYAQKFQGRVTFTVDTSISTAYMELSRLRSDDTGVYFCARLDYRSYWGQGTLVTVSA
(SEQ ID NO: 10)
   Nucleotide sequence of the light chain variable region (4G10L3V): (327 bp) CAGGCTGTCGTCACTCAGGAACCTTCACTGACCGTGTCTCCTGGCGGGACTGTCA 
CCCTGACATGCGGCAGCTCCACAGGGGCCGTGACCACAAGTAACTTCCCAAATTG
GGTCCAGCAGAAGCCAGGACAGGCTCCCCGGAGTCTGATCGGAGGCACCAACAAC
AAGGCCAGCTGGACACCCGCACGGTTCAGCGGCAGCCTGCTGGGCGGCAAGGCC
GCTCTGACAATTAGCGGAGCCCAGCCTGAGGACGAAGCCGAGTACTATTGCGCTCT
GTGGTACTCCAACCACTGGGTGTTCGGCGGCGGCACCAAGCTGACTGTGCTG
(SEQ ID NO: 11)
   The encoded amino acid sequence (4G10L3V): (109 aa)

QAVVTQEPSLTVSPGGTVTLTCGSSTGAVTTSNFPNWVQQKPGQAPRSLIGGTNNKAS
WTPARFSGSLLGGKAALTISGAQPEDEAEYYCALWYSNHWVFGGGTKLTVL (SEQ ID
NO: 12)

### Preparation Example 2: Sequence Design of Anti-PD-1 Antibody 14C12 and its Humanized Antibody 14C12H1L1

The amino acid sequences and encoding nucleotide sequences of the heavy and light chains of the anti-PD-1 antibody 14C12 and its humanized antibodies 14C12H1L1 are identical to those of 14C12 and 14C12H1L1 in Chinese Patent Publication No. CN 106967172A, respectively.
(1) Heavy and light chain variable region sequences of 14C12
   Nucleotide sequence of the heavy chain variable region: (354 bp) 
GAGGTCAAACTGGTGGAGAGCGGCGGCGGGCTGGTGAAGCCCGGCGGGTCACTG
AAACTGAGCTGCGCCGCTTCCGGCTTCGCCTTTAGCTCCTACGACATGTCATGGGT
GAGGCAGACCCCTGAGAAGCGCCTGGAATGGGTCGCTACTATCAGCGGAGGCGGG
CGATACACCTACTATCCTGACTCTGTCAAAGGGAGATTCACAATTAGTCGGGATAAC
GCCAGAAATACTCTGTATCTGCAGATGTCTAGTCTGCGGTCCGAGGATACAGCTCTG
TACTATTGTGCAAACCGGTACGGCGAAGCATGGTTTGCCTATTGGGGACAGGGCAC
CCTGGTGACAGTCTCTGCC (SEQ ID NO: 13)
   The encoded amino acid sequence: (118 aa) 
EVKLVESGGGLVKPGGSLKLSCAASGFAFSSYDMSWVRQTPEKRLEWVATISGGGRYT
YYPDSVKGRFTISRDNARNTLYLQMSSLRSEDTALYYCANRYGEAWFAYWGQGTLVTV
 SA (SEQ ID NO: 14)
   Nucleotide sequence of the light chain variable region: (321 bp) 
GACATTAAGATGACACAGTCCCCTTCCTCAATGTACGCTAGCCTGGGCGAGCGAGT
GACCTTCACATGCAAAGCATCCCAGGACATCAACACATACCTGTCTTGGTTTCAGCA
GAAGCCAGGCAAAAGCCCCAAGACCCTGATCTACCGGGCCAATAGACTGGTGGAC
GGGGTCCCCAGCAGATTCTCCGGATCTGGCAGTGGGCAGGATTACTCCCTGACCAT
CAGCTCCCTGGAGTATGAAGACATGGGCATCTACTATTGCCTGCAGTATGATGAGTT
CCCTCTGACCTTTGGAGCAGGCACAAAACTGGAACTGAAG (SEQ ID NO: 15)
   The encoded amino acid sequence: (107 aa) 
DIKMTQSPSSMYASLGERVTFTCKASQDINTYLSWFQQKPGKSPKTLIYRANRLVDGVP
SRFSGSGSGQDYSLTISSLEYEDMGIYYCLQYDEFPLTFGAGTKLELK (SEQ ID NO: 16)
(2) Heavy and light chain variable region sequences and heavy and light chain sequences of humanized monoclonal antibody 14C12H1L1
   Nucleotide sequence of the heavy chain variable region: (354 bp) 
GAAGTGCAGCTGGTCGAGTCTGGGGGAGGGCTGGTGCAGCCCGGCGGGTCACTG
CGACTGAGCTGCGCAGCTTCCGGATTCGCCTTTAGCTCCTACGACATGTCCTGGGT
GCGACAGGCACCAGGAAAGGGACTGGATTGGGTCGCTACTATCTCAGGAGGCGGG
AGATACACCTACTATCCTGACAGCGTCAAGGGCCGGTTCACAATCTCTAGAGATAAC
AGTAAGAACAATCTGTATCTGCAGATGAACAGCCTGAGGGCTGAGGACACCGCACT
GTACTATTGTGCCAACCGCTACGGGGAAGCATGGTTTGCCTATTGGGGGCAGGGAA
CCCTGGTGACAGTCTCTAGT (SEQ ID NO: 17)
   The encoded amino acid sequence: (118 aa) 
EVQLVESGGGLVQPGGSLRLSCAASGFAFSSYDMSWVRQAPGKGLDWVATISGGGRY
TYYPDSVKGRFTISRDNSKNNLYLQMNSLRAEDTALYYCANRYGEAWFAYWGQGTLVT
VSS (SEQ ID NO: 18)
   Nucleotide sequence of the light chain variable region: (321 bp) 
GACATTCAGATGACTCAGAGCCCCTCCTCCATGTCCGCCTCTGTGGGCGACAGGGT
CACCTTCACATGCCGCGCTAGTCAGGATATCAACACCTACCTGAGCTGGTTTCAGCA
GAAGCCAGGGAAAAGCCCCAAGACACTGATCTACCGGGCTAATAGACTGGTGTCTG
GAGTCCCAAGTCGGTTCAGTGGCTCAGGGAGCGGACAGGACTACACTCTGACCAT
CAGCTCCCTGCAGCCTGAGGACATGGCAACCTACTATTGCCTGCAGTATGATGAGTT
CCCACTGACCTTTGGCGCCGGGACAAAACTGGAGCTGAAG (SEQ ID NO: 19)
   The encoded amino acid sequence: (107 aa) 
DIQMTQSPSSMSASVGDRVTFTCRASQDINTYLSWFQQKPGKSPKTLIYRANRLVSGV
PSRFSGSGSGQDYTLTISSLQPEDMATYYCLQYDEFPLTFGAGTKLELK (SEQ ID NO:
20)
   DNA sequence of 14C12H1L1 heavy chain (14C12H1): (1344 bp) 
GAAGTGCAGCTGGTCGAGTCTGGGGGAGGGCTGGTGCAGCCCGGCGGGTCACTG
CGACTGAGCTGCGCAGCTTCCGGATTCGCCTTTAGCTCCTACGACATGTCCTGGGT
GCGACAGGCACCAGGAAAGGGACTGGATTGGGTCGCTACTATCTCAGGAGGCGGG
AGATACACCTACTATCCTGACAGCGTCAAGGGCCGGTTCACAATCTCTAGAGATAAC
AGTAAGAACAATCTGTATCTGCAGATGAACAGCCTGAGGGCTGAGGACACCGCACT
GTACTATTGTGCCAACCGCTACGGGGAAGCATGGTTTGCCTATTGGGGGCAGGGAA
CCCTGGTGACAGTCTCTAGTGCCAGCACCAAAGGACCTAGCGTGTTTCCTCTCGCC
CCCTCCTCCAAAAGCACCAGCGGAGGAACCGCTGCTCTCGGATGTCTGGTGAAGG
ACTACTTCCCTGAACCCGTCACCGTGAGCTGGAATAGCGGCGCTCTGACAAGCGG
AGTCCATACATTCCCTGCTGTGCTGCAAAGCAGCGGACTCTATTCCCTGTCCAGCGT
CGTCACAGTGCCCAGCAGCAGCCTGGGCACCCAGACCTACATCTGTAACGTCAAC
CACAAGCCCTCCAACACCAAGGTGGACAAGAAAGTGGAGCCCAAATCCTGCGACA
AGACACACACCTGTCCCCCCTGTCCTGCTCCCGAACTCCTCGGAGGCCCTAGCGT
CTTCCTCTTTCCTCCCAAACCCAAGGACACCCTCATGATCAGCAGAACCCCTGAAG
TCACCTGTGTCGTCGTGGATGTCAGCCATGAGGACCCCGAGGTGAAATTCAACTGG
TATGTCGATGGCGTCGAGGTGCACAACGCCAAAACCAAGCCCAGGGAGGAACAGT
ACAACTCCACCTACAGGGTGGTGTCCGTGCTGACAGTCCTCCACCAGGACTGGCT
 
GAACGGCAAGGAGTACAAGTGCAAGGTGTCCAACAAGGCTCTCCCTGCCCCCATT
GAGAAGACCATCAGCAAGGCCAAAGGCCAACCCAGGGAGCCCCAGGTCTATACAC
TGCCTCCCTCCAGGGACGAACTCACCAAGAACCAGGTGTCCCTGACCTGCCTGGT
CAAGGGCTTTTATCCCAGCGACATCGCCGTCGAGTGGGAGTCCAACGGACAGCCC
GAGAATAACTACAAGACCACCCCTCCTGTCCTCGACTCCGACGGCTCCTTCTTCCT
GTACAGCAAGCTGACCGTGGACAAAAGCAGGTGGCAGCAGGGAAACGTGTTCTCC
TGCAGCGTGATGCACGAAGCCCTCCACAACCACTACACCCAGAAAAGCCTGTCCCT
GAGCCCCGGCAAA (SEQ ID NO: 21)
   The encoded amino acid sequence: (448 aa) 
EVQLVESGGGLVQPGGSLRLSCAASGFAFSSYDMSWVRQAPGKGLDWVATISGGGRY
TYYPDSVKGRFTISRDNSKNNLYLQMNSLRAEDTALYYCANRYGEAWFAYWGQGTLVT
VSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO: 22)
   DNA sequence of 14C12H1L1 light chain (14C12L1): (642 bp) 
GACATTCAGATGACTCAGAGCCCCTCCTCCATGTCCGCCTCTGTGGGCGACAGGGT
CACCTTCACATGCCGCGCTAGTCAGGATATCAACACCTACCTGAGCTGGTTTCAGCA
GAAGCCAGGGAAAAGCCCCAAGACACTGATCTACCGGGCTAATAGACTGGTGTCTG
GAGTCCCAAGTCGGTTCAGTGGCTCAGGGAGCGGACAGGACTACACTCTGACCAT
CAGCTCCCTGCAGCCTGAGGACATGGCAACCTACTATTGCCTGCAGTATGATGAGTT
CCCACTGACCTTTGGCGCCGGGACAAAACTGGAGCTGAAGCGAACTGTGGCCGCT
CCCTCCGTCTTCATTTTTCCCCCTTCTGACGAACAGCTGAAATCAGGCACAGCCAG
CGTGGTCTGTCTGCTGAACAATTTCTACCCTAGAGAGGCAAAAGTGCAGTGGAAGG
TCGATAACGCCCTGCAGTCCGGCAACAGCCAGGAGAGTGTGACTGAACAGGACTC
AAAAGATAGCACCTATTCCCTGTCTAGTACACTGACTCTGTCCAAGGCTGATTACGA
 
GAAGCACAAAGTGTATGCATGCGAAGTGACACATCAGGGACTGTCAAGCCCCGTGA
CTAAGTCTTTTAACCGGGGCGAATGT (SEQ ID NO: 23)
   The encoded amino acid sequence: (214 aa)

DIQMTQSPSSMSASVGDRVTFTCRASQDINTYLSWFQQKPGKSPKTLIYRANRLVSGV
PSRFSGSGSGQDYTLTISSLQPEDMATYYCLQYDEFPLTFGAGTKLELKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 24)

### Preparation Example 3: Sequence Design of Bifunctional Antibodies BiAb001(M), BiAb002(M), BiAb003(M) and BiAb004(M)

The structural pattern of the bifunctional antibodies BiAb001(M), BiAb002(M), BiAb003(M), and BiAb004(M) is in the Morrison format (IgG-scFv), i.e., C-termini of two heavy chains of one IgG antibody are linked to scFvs of another antibody, respectively, via linker fragments. The components for the heavy and light chain design are shown in Table A below.

**Table A: Sequence design of BiAb001(M), BiAb002(M), BiAb003(M), and BiAb004(M)**

| Bifunctional antibody | Immunoglobulin portion | | Linker fragment | scFv moiety |
|---|---|---|---|---|
| | Heavy chain | Light chain | | |
| BiAb001(M) | 14C12H1 | 14C12L1 | Linker1 | 4G10H1V(M)- Linker2 - 4G10L1V(M) |
| BiAb002(M) | 14C12H1 | 14C12L1 | Linker2 | 4G10H1V(M)-Linker2 -4G10L1V(M) |
| BiAb003(M) | 14C12H1 | 14C12L1 | Linker1 | 4G10H3V(M)- Linker2 - 4G10L3V(M) |
| BiAb004(M) | 14C12H1 | 14C12L1 | Linker2 | 4G10H3V(M)- Linker2 - 4G10L3V(M) |

| | | | | |
|---|---|---|---|---|
| In Table A above: The amino acid sequence of Linker1 is (GGGGS)3 (SEQ ID NO: 25). The amino acid sequence of Linker2 is (GGGGS)4 (SEQ ID NO: 26). | | | | |

In Table A above, scFv fragments 4G10H1V(M), 4G10L1V(M), 4G10H3V(M), and 4G10L3V(M) of BiAb001(M), BiAb002(M), BiAb003(M), and BiAb004(M) antibodies comprise mutations in individual amino acids of framework regions on the basis of 4G10H1V, 4G10L1V, 4G10H3V, and 4G10L3V, respectively, which effectively optimizes the structure of the antibodies and improves their efficacy.
(1) 4G10H1V(M): (115 aa, mutation sites underlined in the amino acid sequence based on 4G10H1V) 
QVQLVESGAELVKPGASMKISCKASGYSFTGYTMNWVKQAPGQCLEWIGLINPYNNIT
NYNQKFMGKATFTVDKSISTAYMELSRLTSDDSGVYFCARLDYRSYWGQGTLVTVSA
(SEQ ID NO: 41)
(2) 4G10L1V(M): (110 aa, mutation sites underlined in the amino acid sequence based on 4G10L1V) 
QAVVTQEPSLTVSPGGTVTLTCGSSTGAVTTSNFANWVQEKPGQAFRSLIGGTNNRAS
WVPARFSGSLLGGKAALTISGAQPEDEAEYFCALWYSNHWVFGCGTKLTVLR (SEQ ID
NO: 42)
(3) 4G10H3V(M): (115 aa, mutation sites underlined in the amino acid sequence based on 4G10H3V) 
QVQLVESGAEVKKPGASVKVSCKASGYSFTGYTMNWVRQAPGQCLEWIGLINPYNNIT
NYAQKFQGRVTFTVDTSISTAYMELSRLRSDDTGVYFCARLDYRSYWGQGTLVTVSA
(SEQ ID NO: 43)
(4) 4G10L3V(M): (110 aa, mutation sites underlined in the amino acid sequence based on 4G10L3V)

QAVVTQEPSLTVSPGGTVTLTCGSSTGAVTTSNFPNWVQQKPGQAPRSLIGGTNNKAS
WTPARFSGSLLGGKAALTISGAQPEDEAEYYCALWYSNHWVFGCGTKLTVLR (SEQ ID
NO: 44)

For distinguishment from the mutated antibodies hereinafter, BiAb004(M) is also referred to as BiAb004(hG1WT) in the examples of the present disclosure. BiAb004(M) described above is the "wild-type", comprising an Ig gamma-1 chain C region (ACCESSION: P01857) as the heavy chain constant region and an Ig kappa chain C region (ACCESSION: P01834) as the light chain constant region.

### Preparation Example 4: Non-Variable Region Amino Acid Mutation Design Based on Humanized Bifunctional Antibody BiAb004

On the basis of BiAb004(hG1WT) obtained in Preparation Example 3, BiAb004(hG1TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain by the inventors.
DNA sequence of heavy chain of the immunoglobulin portion in BiAb004(hG1TM): (1344 bp, mutation sites underlined) 
GAAGTGCAGCTGGTCGAGTCTGGGGGAGGGCTGGTGCAGCCCGGCGGGTCACTG
CGACTGAGCTGCGCAGCTTCCGGATTCGCCTTTAGCTCCTACGACATGTCCTGGGT
GCGACAGGCACCAGGAAAGGGACTGGATTGGGTCGCTACTATCTCAGGAGGCGGG
AGATACACCTACTATCCTGACAGCGTCAAGGGCCGGTTCACAATCTCTAGAGATAAC
AGTAAGAACAATCTGTATCTGCAGATGAACAGCCTGAGGGCTGAGGACACCGCACT
GTACTATTGTGCCAACCGCTACGGGGAAGCATGGTTTGCCTATTGGGGGCAGGGAA
CCCTGGTGACAGTCTCTAGTGCCAGCACCAAAGGGCCCAGCGTGTTTCCTCTCGC
CCCCTCCTCCAAAAGCACCAGCGGAGGAACCGCTGCTCTCGGATGTCTGGTGAAG
GACTACTTCCCTGAACCCGTCACCGTGAGCTGGAATAGCGGCGCTCTGACAAGCG
GAGTCCATACATTCCCTGCTGTGCTGCAAAGCAGCGGACTCTATTCCCTGTCCAGC
GTCGTCACAGTGCCCAGCAGCAGCCTGGGCACCCAGACCTACATCTGTAACGTCAA
CCACAAGCCCTCCAACACCAAGGTGGACAAGAAAGTGGAGCCCAAATCCTGCGAC
AAGACACACACCTGTCCCCCCTGTCCTGCTCCCGAAGCTGCTGGAGCCCCTAGCG
TCTTCCTCTTTCCTCCCAAACCCAAGGACACCCTCATGATCAGCAGAACCCCTGAA
GTCACCTGTGTCGTCGTGGATGTCAGCCATGAGGACCCCGAGGTGAAATTCAACTG
GTATGTCGATGGCGTCGAGGTGCACAACGCCAAAACCAAGCCCAGGGAGGAACAG
TACAACTCCACCTACAGGGTGGTGTCCGTGCTGACAGTCCTCCACCAGGACTGGCT
GAACGGCAAGGAGTACAAGTGCAAGGTGTCCAACAAGGCTCTCCCTGCCCCCATT
 
GAGAAGACCATCAGCAAGGCCAAAGGCCAACCCAGGGAGCCCCAGGTCTATACAC
TGCCTCCCTCCAGGGACGAACTCACCAAGAACCAGGTGTCCCTGACCTGCCTGGT
CAAGGGCTTTTATCCCAGCGACATCGCCGTCGAGTGGGAGTCCAACGGACAGCCC
GAGAATAACTACAAGACCACCCCTCCTGTCCTCGACTCCGACGGCTCCTTCTTCCT
GTACAGCAAGCTGACCGTGGACAAAAGCAGGTGGCAGCAGGGAAACGTGTTCTCC
TGCAGCGTGATGCACGAAGCCCTCCACAACCACTACACCCAGAAAAGCCTGTCCCT
GAGCCCCGGCAAA (SEQ ID NO: 39)
Amino acid sequence of heavy chain of the immunoglobulin portion in BiAb004(hG1TM): (448 aa, mutation sites underlined) 
EVQLVESGGGLVQPGGSLRLSCAASGFAFSSYDMSWVRQAPGKGLDWVATISGGGRY
TYYPDSVKGRFTISRDNSKNNLYLQMNSLRAEDTALYYCANRYGEAWFAYWGQGTLVT
VSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAP
EAAGAPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO: 40)
 BiAb004(hG1TM) and BiAb004(hG1WT) share the same DNA sequence of light chain and the same encoded amino acid sequence. The specific sequence is shown in Preparation Example 3.

### Example 1: Assay for Biological Activity of Anti-CTLA4-Anti-PD-1 Bispecific Antibody in Combination with Gemcitabine and Paclitaxel in Promoting IFN-γ Secretion by Mixed Lymphocyte Reaction (MLR)

1. PBMC treatment: Normal human PBMCs were isolated according to the Ficoll-Paque^{™} Plus isolation solution instruction. Two days before the experiment, PBMCs were thawed and cultured in a complete medium in a 5% carbon dioxide incubator at 37 °C. After 2 h, when the state of the PBMCs was recovered, SEB (*Staphylococcus aureus* enterotoxin antigen, at a final concentration of 0.5 µg/mL) was added for stimulation for two days.
2. BxPC-3 treatment: Two days before the experiment, human pancreatic cancer cells BxPC-3 (purchased from Shanghai Institutes for Biological Sciences, Chinese Academy of Sciences) were conventionally collected and centrifuged at 170× g for 5 min. 3 × 10⁶ BxPC-3 cells were seeded into culture dishes and divided into 4 groups according to the following experimental design:
   (1) no treatment (as a negative control);
   (2) adding gemcitabine to a final concentration of 100 nM and treating for 48 h;
   (3) adding albumin-bound paclitaxel to a final concentration of 20 ng/mL and treating for 24 h; and
   (4) adding gemcitabine to a final concentration of 100 nM and treating for 24 h; and then adding albumin-bound paclitaxel to a final concentration of 20 ng/mL and treating for another 24 h.
3. Raji-PDL1 treatment: On the day of the experiment, Raji-PDL1 cells were collected, centrifuged at 110× g for 5 min, resuspended (in an assay medium 1640 + 10% FBS), and counted. The cell density was adjusted to 2,000,000 cells/mL, and MMC (Mito-mycin C, at a final concentration of 2 µg/mL, purchased from Stressmarq, Cat. No. SIH-246-10MG) was added. The cells were treated in a 5% carbon dioxide incubator at 37 °C for 1 h.
4. PBMCs after two days of SEB stimulation and Raji-PDL1 cells after MMC treatment were collected, washed twice with the assay medium, resuspended, and counted; BxPC-3 cells treated with different chemotherapeutic drugs/without treatment in step 2 were conventionally collected, centrifuged at 170× g for 5 min, resuspended, and counted; the cells were seeded into a 96-well U-shaped plate (3799) according to PBMCs at 100,000 cells/well, Raji-PDL1 at 100,000 cells/well, and BxPC-3 at 20,000 cells/well to obtain a mixed lymphocyte system.
5. The antibodies were added according to the following groups:
   BiAb004(hG1TM) at final concentrations of 0.3/3/30 nM in the system was separately added to the BiAb004(hG1TM) group, the gemcitabine + BiAb004(hG1TM) group, and the gemcitabine + albumin-bound paclitaxel + BiAb004(hG1TM) group.
   Opdivo at final concentrations of 0.3/3/30 nM and Yervoy at final concentrations of 0.3/3/30 nM were separately added to the gemcitabine + Opdivo + Yervoy group and the gemcitabine + albumin-bound paclitaxel + Opdivo + Yervoy group.
   No antibody was added to the negative control group (PBMC + Raji-PDL1 + BxPC-3 without chemotherapeutic drug treatment), the gemcitabine treatment alone group, the albumin-bound paclitaxel (Abraxane) treatment alone group, and the gemcitabine + albumin-bound paclitaxel (gemcitabine + Abraxane) treatment group.
   The final volume of the system in each group was 200 µL, and the cells were cultured for 3 days.
6. After 3 days, the cells were centrifuged at 250× g for 5 min (Beckman centrifuge), and cell supernatants were collected, and the IFN-γ content was assayed by using Dakewe kit.

The results are shown in FIG. 1.

The results show that in the mixed lymphocyte system, the ability of the anti-CTLA4-anti-PD-1 bispecific antibody BiAb004(hG1TM) + gemcitabine combination group to promote INF-γ was stronger than those of the BiAb004(hG1TM) single-drug group and the anti-PD-1 monoclonal antibody Opdivo (purchased from BMS, batch No. ABS3203) + anti-CTLA4 monoclonal antibody Yervoy (purchased from BMS, batch No. AAT3892) + gemcitabine combination group, and the ability of the BiAb004(hG1TM) + gemcitabine + albumin-bound paclitaxel combination group to promote INF-γ was stronger than that of the anti-PD-1 monoclonal antibody Opdivo + anti-CTLA4 monoclonal antibody Yervoy + gemcitabine + albumin-bound paclitaxel combination group.

Although the specific embodiments of the present disclosure have been described in detail, those skilled in the art will appreciate that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. A therapeutic combination, comprising an anti-CTLA4-anti-PD-1 bispecific antibody and gemcitabine or a pharmaceutically acceptable salt thereof, wherein
preferably, the therapeutic combination further comprises paclitaxel.

2. The therapeutic combination according to claim 1, wherein the pharmaceutically acceptable salt of gemcitabine is gemcitabine hydrochloride.

3. The therapeutic combination according to any one of claims 1 to 2, wherein paclitaxel is albumin-bound paclitaxel;
preferably, paclitaxel and albumin are in a mass ratio of 1:(5-15), 1:(8-12), 1:8, 1:9, 1:10, 1:11, or 1:12.

4. The therapeutic combination according to any one of claims 1 to 3, wherein the anti-CTLA4-anti-PD-1 bispecific antibody is in a unit dose of 100 mg-1000 mg, 200 mg-800 mg, 200 mg-500 mg, 300 mg-600 mg, 400 mg-500 mg, or 450 mg.

5. The therapeutic combination according to any one of claims 1 to 4, wherein the anti-CTLA4-anti-PD-1 bispecific antibody is administered at a unit dose of 0.1-100 mg, preferably 1-15 mg, 1-12 mg, 1-10 mg, or 6-10 mg, per kg body weight.

6. The therapeutic combination according to any one of claims 1 to 5, wherein:
based on the mass (mg) of gemcitabine or the pharmaceutically acceptable salt thereof/body surface area (m²) of a subject, gemcitabine or the pharmaceutically acceptable salt thereof is administered at a unit dose of 500-1500 mg/m², 600-1300 mg/m², 800-1250 mg/m², 800-1000 mg/m², 800 mg/m², 850 mg/m², 900 mg/m², 950 mg/m², 1000 mg/m², 1050 mg/m², 1100 mg/m², 1150 mg/m², 1200 mg/m², or 1250 mg/m²; or
gemcitabine or the pharmaceutically acceptable salt thereof is in a unit dose of 100-2000 mg, 500-1500 mg, 600-1300 mg, 800-1200 mg, 800-1000 mg, 800 mg, 900 mg, 1000 mg, 1100 mg, 1200 mg, or 1500 mg.

7. The therapeutic combination according to any one of claims 1 to 6, wherein:
based on the mass (mg) of albumin-bound paclitaxel/body surface area (m²) of a subject, albumin-bound paclitaxel is administered at a unit dose of 80-300 mg/m², 100-280 mg/m², 120-260 mg/m², 120-200 mg/m², 120-180 mg/m², 125-175 mg/m², 125 mg/m², 130 mg/m², 135 mg/m², 140 mg/m², 145 mg/m², 150 mg/m², 155 mg/m², 160 mg/m², 165 mg/m², 170 mg/m², 175 mg/m², or 260 mg/m²; or
albumin-bound paclitaxel is in a unit dose of 200-2000 mg, 500-1500 mg, 800-1200 mg, 900-1100 mg, 800 mg, 900 mg, 1000 mg, 1100 mg, or 1200 mg.

8. The therapeutic combination according to any one of claims 1 to 7, wherein
the therapeutic combination is a fixed combination, e.g., a pharmaceutical composition;
the therapeutic combination is a non-fixed combination, e.g., the anti-CTLA4-anti-PD-1 bispecific antibody, gemcitabine or the pharmaceutically acceptable salt thereof, and paclitaxel in the non-fixed combination are each present in a separate pharmaceutical composition; or
gemcitabine or the pharmaceutically acceptable salt thereof and paclitaxel are in a fixed combination, e.g., in a pharmaceutical composition; the anti-CTLA4-anti-PD-1 bispecific antibody is present in a separate pharmaceutical composition.

9. The therapeutic combination according to claim 8, wherein the pharmaceutical composition is independently a solid pharmaceutical composition or a liquid pharmaceutical composition;
preferably, the pharmaceutical composition further comprises one or more pharmaceutically acceptable auxiliary materials;
preferably, the pharmaceutical composition does not comprise an active pharmaceutical ingredient other than the anti-CTLA4-anti-PD-1 bispecific antibody, gemcitabine or the pharmaceutically acceptable salt thereof, and paclitaxel.

10. The therapeutic combination according to any one of claims 1 to 9, wherein the anti-CTLA4-anti-PD-1 bispecific antibody comprises:
a first protein functional region targeting PD-1 and
a second protein functional region targeting CTLA4,
wherein:
the first protein functional region is an immunoglobulin, and the second protein functional region is a single chain fragment variable, wherein a heavy chain variable region of the immunoglobulin comprises HCDR1-HCDR3 having amino acid sequences set forth in SEQ ID NOs: 27-29, respectively, and a light chain variable region of the immunoglobulin comprises LCDR1-LCDR3 having amino acid sequences set forth in SEQ ID NOs: 30-32, respectively; a heavy chain variable region of the single chain fragment variable comprises HCDR1-HCDR3 having amino acid sequences set forth in SEQ ID NOs: 33-35, respectively, and a light chain variable region of the single chain fragment variable comprises LCDR1-LCDR3 having amino acid sequences set forth in SEQ ID NOs: 36-38, respectively;
or
the first protein functional region is a single chain fragment variable, and the second protein functional region is an immunoglobulin, wherein a heavy chain variable region of the single chain fragment variable comprises HCDR1-HCDR3 having amino acid sequences set forth in SEQ ID NOs: 27-29, respectively, and a light chain variable region of the single chain fragment variable comprises LCDR1-LCDR3 having amino acid sequences set forth in SEQ ID NOs: 30-32, respectively; a heavy chain variable region of the immunoglobulin comprises HCDR1-HCDR3 having amino acid sequences set forth in SEQ ID NOs: 33-35, respectively, and a light chain variable region of the immunoglobulin comprises LCDR1-LCDR3 having amino acid sequences set forth in SEQ ID NOs: 36-38, respectively;
preferably, the immunoglobulin is of human IgG1 subtype, and according to the EU numbering system, a heavy chain constant region of the immunoglobulin comprises one of the following combinations of mutations:
L234A and L235A; or
L234A and G237A; or
L235A and G237A; or
L234A, L235A, and G237A.

11. The therapeutic combination according to any one of claims 1 to 10, wherein the anti-CTLA4-anti-PD-1 bispecific antibody is provided, wherein according to the EU numbering system, the heavy chain constant region of the immunoglobulin further comprises one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

12. The therapeutic combination according to any one of claims 1 to 11, wherein the anti-CTLA4-anti-PD-1 bispecific antibody is provided, wherein
an amino acid sequence of the heavy chain variable region of the immunoglobulin is selected from SEQ ID NO: 14 and SEQ ID NO: 18, and an amino acid sequence of the light chain variable region of the immunoglobulin is selected from SEQ ID NO: 16 and SEQ ID NO: 20; an amino acid sequence of the heavy chain variable region of the single chain fragment variable is selected from SEQ ID NO: 2, SEQ ID NO: 6, SEQ ID NO: 10, SEQ ID NO: 41 and SEQ ID NO: 43, and an amino acid sequence of the light chain variable region of the single chain fragment variable is selected from SEQ ID NO: 4, SEQ ID NO: 8, SEQ ID NO: 12, SEQ ID NO: 42 and SEQ ID NO: 44;
or
an amino acid sequence of the heavy chain variable region of the immunoglobulin is selected from SEQ ID NO: 2, SEQ ID NO: 6, SEQ ID NO: 10, SEQ ID NO: 41 and SEQ ID NO: 43, and an amino acid sequence of the light chain variable region of the immunoglobulin is selected from SEQ ID NO: 4, SEQ ID NO: 8, SEQ ID NO: 12, SEQ ID NO: 42 and SEQ ID NO: 44; an amino acid sequence of the heavy chain variable region of the single chain fragment variable is selected from SEQ ID NO: 14 and SEQ ID NO: 18, and an amino acid sequence of the light chain variable region of the single chain fragment variable is selected from SEQ ID NO: 16 and SEQ ID NO: 20.

13. The therapeutic combination according to any one of claims 1 to 12, wherein the anti-CTLA4-anti-PD-1 bispecific antibody is selected from any one of the following (1)-(20):
(1)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(2)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(3)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(4)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(5)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(6)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(7)
an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(8)
an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(9)
an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(10)
an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(11)
an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(12)
an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(13)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(14)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(15)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(16)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(17)
an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(18)
an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(19)
an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
and
(20)
an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 18, and an amino acid sequence of the light chain variable region of the single chain fragment variable is set forth in SEQ ID NO: 20.

14. The therapeutic combination according to any one of claims 1 to 13, wherein the anti-CTLA4-anti-PD-1 bispecific antibody is provided:
an amino acid sequence of the heavy chain of the immunoglobulin is set forth in SEQ ID NO: 40, and an amino acid sequence of the light chain is set forth in SEQ ID NO: 24.

15. The therapeutic combination according to any one of claims 1 to 14, wherein the anti-CTLA4-anti-PD-1 bispecific antibody is provided, wherein the first protein functional region is linked, either directly or via a linker fragment, to the second protein functional region, and/or the heavy chain variable region of the single chain fragment variable is linked, either directly or via a linker fragment, to the light chain variable region of the single chain fragment variable;
preferably, the linker fragment is a polypeptide set forth in SEQ ID NO: 45, or a polypeptide consisting of a plurality of (e.g., 2, 3, 4, 5, or 6) polypeptides set forth in SEQ ID NO: 45 linked in series.

16. The therapeutic combination according to any one of claims 1 to 15, wherein the anti-CTLA4-anti-PD-1 bispecific antibody is provided, wherein the numbers of the first protein functional region and the second protein functional region are each independently 1, 2, or more.

17. The therapeutic combination according to any one of claims 1 to 16, wherein the anti-CTLA4-anti-PD-1 bispecific antibody is provided, wherein the single chain fragment variables are linked to the C-termini of two heavy chains of the immunoglobulin, respectively.

18. The therapeutic combination according to any one of claims 1 to 17, wherein the anti-CTLA4-anti-PD-1 bispecific antibody comprises:
a first protein functional region targeting PD-1 and
a second protein functional region targeting CTLA4,
the first protein functional region is 1 in number, and the second protein functional region is 2 in number;
wherein the first protein functional region is an immunoglobulin, and the second protein functional region is a single chain fragment variable;
an amino acid sequence of the heavy chain of the immunoglobulin is set forth in SEQ ID NO: 40, and an amino acid sequence of the light chain is set forth in SEQ ID NO: 24;
an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
the single chain fragment variable is linked to the C-terminus of the heavy chain of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NO: 25 and SEQ ID NO: 26;
preferably, amino acid sequences of the first linker fragment and the second linker fragment are set forth in SEQ ID NO: 26.

19. A kit product, comprising the therapeutic combination according to any one of claims 1 to 18, and a package insert.

20. Use of the therapeutic combination according to any one of claims 1 to 18 in preparing a medicament for treating or preventing a tumor, wherein
preferably, the tumor is selected from one or more of pancreatic cancer, melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the pancreatic cancer is selected from pancreatic ductal adenocarcinoma and pancreatic adenosquamous carcinoma;
preferably, the pancreatic cancer is advanced or metastatic pancreatic cancer; preferably, the lung cancer is selected from one or more of non-small cell lung cancer, small cell lung cancer and squamous cell lung cancer;
preferably, the non-small cell lung cancer is intermediate or advanced non-small cell lung cancer;
preferably, the gastric cancer is gastric adenocarcinoma or gastroesophageal junction adenocarcinoma.

21. The therapeutic combination according to any one of claims 1 to 18 for use in the treatment or prevention of a tumor, wherein
preferably, the tumor is selected from one or more of pancreatic cancer, melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the pancreatic cancer is selected from pancreatic ductal adenocarcinoma and pancreatic adenosquamous carcinoma;
preferably, the pancreatic cancer is advanced or metastatic pancreatic cancer;
preferably, the lung cancer is selected from one or more of non-small cell lung cancer, small cell lung cancer and squamous cell lung cancer;
preferably, the non-small cell lung cancer is intermediate or advanced non-small cell lung cancer;
preferably, the gastric cancer is gastric adenocarcinoma or gastroesophageal junction adenocarcinoma.

22. A method for treating or preventing a tumor, comprising a step of administering to a subject in need an effective amount of the therapeutic combination according to any one of claims 1 to 18, wherein
preferably, the tumor is selected from one or more of pancreatic cancer, melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the pancreatic cancer is selected from pancreatic ductal adenocarcinoma and pancreatic adenosquamous carcinoma;
preferably, the pancreatic cancer is advanced or metastatic pancreatic cancer; preferably, the lung cancer is selected from one or more of non-small cell lung cancer, small cell lung cancer and squamous cell lung cancer;
preferably, the non-small cell lung cancer is intermediate or advanced non-small cell lung cancer;
preferably, the gastric cancer is gastric adenocarcinoma or gastroesophageal junction adenocarcinoma.
